# EUROPÄISCHE PATENTANMELDUNG

(11) **EP 1 312 299 A1**
(43) Veröffentlichungstag der Anmeldung: **21.05.2003**
(21) Anmeldenummer: 01811101.3
(22) Anmeldetag: 16.11.2001
(51) Int. Cl.: A61B 3/12, A61B 3/00

(54) **Vorrichtung zur Betrachtung des Auges**

(71) Anmelder: Mojon, Daniel, 9008 St. Gallen (CH)
(72) Erfinder: Mojon, Daniel, 9008 St. Gallen (CH)
(74) Vertreter: Roshardt, Werner Alfred, Dipl.-Phys.

(57) **Zusammenfassung**

Die Vorrichtung **(1)** zur Betrachtung und Beleuchtung des Auges, insbesondere des Augenfundus **(3)** hat eine Beleuchtungseinheit **(20a/b, 11a/b; 23a, 23b)** und eine vor dem zu untersuchenden Patientenauge **(5)** zu plazierende Lupenlinseneinheit **(7).** Die Beleuchtungseinheit **(20a/b, 11a/b; 23a, 23b)** ist derart ausgebildet und angeordnet, dass aus ihr austretende Freiraumstrahlung **(9a, 9b)** in der Lupenlinseneinheit **(7)** nicht in den Beobachtungsstrahlengang **(10)** fällt. Die Vorrichtung ist einfach zu handhaben. Störende Reflexe des Beleuchtungsstrahlenganges an den Oberflächen der für die Betrachtung notwendigen optischen Komponenten konnten vermieden werden.

## Beschreibung

### Technisches Gebiet

Die Erfindung betrifft eine Vorrichtung zur Betrachtung und Beleuchtung des Auges, insbesondere des Augenfundus, mit einer Lupenlinse.

### Stand der Technik

In der DE-A 31 24 305 wird ein Handophthalmoskop zur Beobachtung des Augenhintergrundes beschrieben. Das Handophthalmoskop hat einen seitlich eingeblendeten Beleuchtungsstrahl, der über einen kleinen Spiegel [45°-Prisma] unmittelbar vor der dem Auge zugewandten Lupenlinsenoberfläche in den Beobachtungsstrahlengang eingekoppelt wird. Im Beobachtungsstrahlengang ist ein Bildverstärkerelement angeordnet, um mit kleinen Lichtstärken arbeiten zu können. Infolge der kleinen Lichtstärken bleibt nämlich der Pupillendurchmesser des Patienten während der Beobachtung groß.

Im deutschen Gebrauchsmuster DE-GM 72 10 865 ist ein Binokular-Ophthalmoskop mit einem Lampenstab beschrieben. Die optische Achse des Lampenstabes ist zur Beobachtungsachse unter einem spitzen Winkel angeordnet. Der Beleuchtungsstrahlengang verläuft zur optischen Achse des Beobachtungsstrahlenganges leicht versetzt und gekippt. Die Einkopplung des Beleuchtungsstrahls erfolgt über eine spiegelnde Fläche auf der dem Auge abgewandten Seite der Lupenlinse.

### Darstellung der Erfindung

### Aufgabe

Aufgabe der Erfindung ist es, eine einfach zu handhabende Vorrichtung zur Betrachtung des Auges, insbesondere des Augenfundus zu schaffen, bei der störende Reflexe des Beleuchtungsstrahlenganges an den Oberflächen der für die Betrachtung notwendigen optischen Komponenten vermieden werden.

### Lösung

Die Lösung der Aufgabe erfolgt durch die Merkmale des Anspruchs 1. Lupenlinseneinheit und Beleuchtungseinheit bilden eine Gesamteinheit. Im Gegensatz zum Stand der Technik fallen Beleuchtungs- und Beobachtungsstrahl in der Lupenlinseneinheit nicht mehr zusammen; d.h. die beiden Strahlengänge laufen an diesem Ort getrennt voneinander. Es können somit in den Beobachtungsstrahl von Oberflächen optischer Elemente der Lupenlinseneinheit keine Reflexionen mehr in den Beobachtungsstrahlengang gelangen.

Vorzugsweise wird man die Beleuchtungseinheit derart ausbilden und anordnen, dass ihre Freiraumstrahlung nicht mehr durch die Lupenlinseneinheit verläuft.

Dies kann beispielsweise dadurch erfolgen, dass der Beleuchtungsstrahlengang derart gelegt wird, dass er ausserhalb des Beobachtungsstrahlenganges verläuft. D. h. man wird ihn in den Randbereich der Lupenlinse bzw. in die diesem benachbarte Umgebung legen.

Die Beleuchtungsstrahlung kann nun in einen Bereich ausserhalb des Beobachtungsstrahlenganges mittels Lichtquellen direkt erzeugt werden oder mittels Strahlungs- bzw. Lichtleitern dort hingebracht werden. Um zwischen der Beleuchtungsstrahlung in Lichtleitern und der Beleuchtungsstrahlung, welche auf das Auge bzw. in dieses hineingerichtet wird, unterscheiden zu können, wird der Ausdruck Freiraumstrahlung verwendet. Freiraumstrahlung ist jegliche Strahlung, welche durch den freien Raum verläuft. Hierunter fällt Strahlung, welche von einer Strahlungsquelle ausgesandt wird und nicht durch Strahlungsleiter geführt wird. Eine Freiraumstrahlung ist auch die Strahlung, welche aus einem Lichtleiter ausgetreten ist und dann beispielsweise durch Linsen- oder Spiegelsysteme geformt wird.

Die Freiraumstrahlung zur Beleuchtung des Auges bzw. des Augenfundus wird nun durch optische Systeme derart geformt und gerichtet, dass sie in den gewünschten Bereich kommt und die gewünschte Fläche beleuchtet.

Um eine derartige Strahlführung zu erreichen, ist mit optischen Systemen der Fokussierkegel entsprechend einzustellen bzw. die optische Achse der Beleuchtungsstrahlung entsprechend auszurichten. Eine Einstellung erfolgt, indem die örtliche Lage der Strahlungsquelle bzw. das Ende von Strahlungsleitern in bezug auf das optische System verändert wird. Eine Veränderung kann aber auch erreicht werden, indem das optische System geändert wird, indem Linsen oder Hohlspiegel verschoben bzw. verformt werden.

Hierzu kann man beispielsweise die Lichtleiterenden bzw. die Strahlungsquellen (z. B. LED) in einem elastischen Mittel z. B. einem Band anordnen, welches dann gegenüber dem optischen System verschoben wird.

Wie oben bereits angedeutet, können aktive Strahlungsquellen direkt ausserhalb des Beobachtungsstrahlenganges angeordnet werden. Sie können aber auch "weit" entfernt liegen (z. B. in einem Handgriff, mit dem die Lupenlinse gehalten werden kann) und über Lichtleiter an den betreffenden Ort gebracht werden.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine schematische Darstellung der erfindungsgemässen Vorrichtung zur Betrachtung und Beleuchtung des Augenfundus,
- Fig. 2: eine Draufsicht auf die Lupenlinseneinheit mit einer schematisch dargestellten ringförmigen Anordnung von Beleuchtungsstrahlung aussendenden Elementen, wobei eine Halterung für die Lichtquellen nicht und die optische Strahlformung stark vereinfacht dargestellt sind, und
- Fig. 3: eine Ansicht vom Patientenauge aus gegen die Lupenlinse analog zur Darstellung in Figur 2, bei der eine Verstellung der Beleuchtungsstrahlung aussendenden Elemente angedeutet ist.

Grundsätzlich sind in den Figuren gleiche Teile mit gleichen Bezugszeichen versehen.

### Wege zur Ausführung der Erfindung

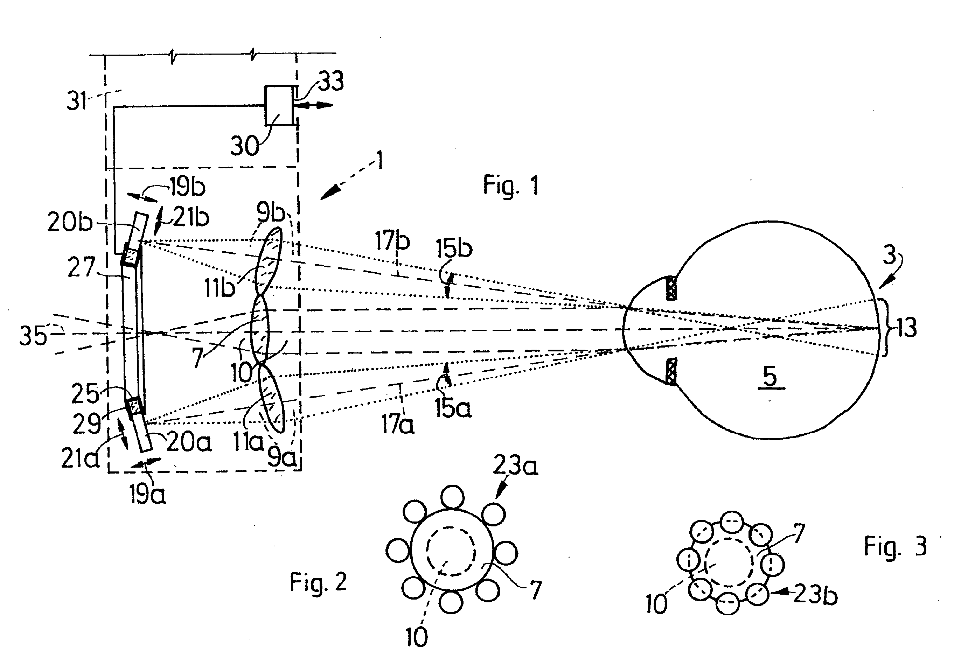

Die in Figur **1** dargestellte erfindungsgemässe Vorrichtung **1** zur Betrachtung und Beleuchtung des Fundus **3** eines Patientenauges **5** hat eine Lupenlinse **7** als Lupenlinseneinheit. Die Lupenlinse **7** wird bei einer Untersuchung vor dem zu untersuchenden Auge **5** plaziert. Die Beleuchtungseinheit ist derart ausgebildet und angeordnet, dass aus ihr austretende Freiraumstrahlung **9a** und **9b** in der Lupenlinse **7** nicht in den Beobachtungsstrahlengang **10** fällt. Die Lupenlinse **7** wird von der Beleuchtungsstrahlung **9a** und **9b** nicht durchstrahlt. Die Beleuchtungsstrahlung **9a** und **9b** verläuft hier beispielsweise als Freiraumstrahlung **9a** und **9b** ausserhalb der Lupenlinse **7**.

Die Freiraumstrahlung **9a** und **9b** wird mit einer Fokussiereinheit, welche hier symbolisch durch die beiden Fokussierlinsen **11a** und **11b** dargestellt ist, derart geformt, dass ein vorgegebener Bereich **13** auf dem Fundus **3** beleuchtet wird.

Der mit den Fokussierlinsen **11a** und **11b** erzeugbare Fokussierkegelwinkel **15a** und **15b** kann durch eine Relativbewegung jeder Lichtquelle entlang der optischen Achse **17a** bzw. **17b** auf die Fokussierlinse **11a** bzw. **11b** zu oder von dieser weg, entprechend der Doppelpfeile **19a** bzw. **19b,** verändert werden. Als Lichtquelle sind hier beispielsweise je ein LED (Licht (Strahlung) emittierende Diode) **20a** bzw. **20b** dargestellt; Varianten hierzu sind unten erwähnt. Werden die LED **20a** und **20b** gemäss der Doppelpfeile **21a** und **21b** verschwenkt, erfolgt auch eine Verschwenkung der Freiraumstrahlen **9a** und **9b.**

Anstatt die LED **20a** und **20b** zu verschieben, können selbstverständlich auch die Fokussierlinsen **11a** und **11b** relativ zu den LEDs **20a** und **20b** verschoben werden.

Um eine möglichst gute Ausleuchtung des Fundus **3** zu erreichen, wird man nicht nur die beiden in **Figur 1** dargestellten Lichtquellen **20a** und **20b** verwenden, obwohl in vielen Fällen bereits lediglich eine Lichtquelle ausreichen sollte, sondern wie in den Figuren **2** und **3** gezeigt, eine ringförmige Anordnung **23a** bzw. **23b** mit einer Vielzahl von LEDs. Die Anordnung **23a** bzw. **23b** wird man derart vornehmen, dass die aus den LEDs austretende Beleuchtungsstrahlung **9a** und **9b** im Bereich der Lupenlinse **7** nicht in den Beobachtungsstrahlengang **10** fällt. Vorzugsweise wird man die Anordnung **23a** bzw. **23b** in unmittelbarer Nähe zur Lupenlinsenoberfläche anbringen, die dem Patientenauge **5** zugewandt ist.

Die LEDs der Anordnung **23a** bzw. **23b** können in einer Ausführungsvariante an der Innenwand **25** eines in einer U-förmigen Halterung **27** liegenden kreisförmigen Schlauchs **29** angeordnet werden. Der Schlauchmantel besteht aus elastischem Material. Der Schlauch **29** ist mit einer Flüssigkeit gefüllt, welche aus einer Speichereinheit **30** in den Schlauchinnenraum drückbar ist. Wird Flüssigkeit in den Schlauchinnenraum gedrückt, wandert dessen Innenwand **25** radial nach aussen und schiebt gleichzeitig die LEDs radial nach aussen. Wird Flüssigkeit abgezogen, erfolgt eine radiale Bewegung nach innen. Es ist somit eine Bewegung der LEDs entsprechend der Doppelpfeile **21a** und **21b** möglich. Durch diese Bewegung ergibt sich dann die oben erwähnte Verschwenkung der optischen Achse **17a** bzw. **17b.** Ein Verkippen der LEDs beim Aufblähen bzw. Schrumpfen des Schlauches **29** ist ausgeschlossen, da die LEDs durch die Schenkel der U-förmigen Halterung **27** geführt sind.

Die Lichtquellen **20a** und **20b** werden in **Figur 1** gemäss den zueinander senkrecht stehenden Richtungspfeilen **19a/b** und **21a/b** verschoben. Anstelle dieser Verschieberichtungen sind selbstverständlich auch Schiebungen entlang einer Kurve möglich. Die Schenkel der Halterung **27** sind dann entsprechend zu formen.

Die Speichereinheit **30** kann in einem zur Vorrichtung **1** gehörenden Haltegriff **31** untergebracht werden. Ein Einpressen bzw. Abziehen von Flüssigkeit aus dem Schlauch **29** erfolgt dann, wie angedeutet, durch Betätigen eines vorzugsweise federbelasteten Druckknopfes **33**.

Zum Verstellen des Fokussierkegels **15a** bzw. **15b** wird die U-förmige Halterung **27** mit den LEDs parallel zur optischen Achse **35** des Beobachtungsstrahlenganges **10** verschoben. Diese Verschiebung bewirkt neben der Veränderung des Fokussierkegels **15a** und **15b** auch eine Verschwenkung der optischen Achse **17a** bzw. **17b.** Diese Verschwenkung kann durch eine entsprechende Betätigung des Einstellknopfes **33** korrigiert werden. Vorzugsweise wird man jedoch beide Verstellungen selbsthemmend ausbilden, wobei die zweitgenannte Verstellung über eine getriebeartige Übersetzung auch auf die Flüssigkeitszufuhr zum Schlauch **29** wirkt, um eine entsprechende Korrektur vorzunehmen.

Die Verstellung der LEDs **20a, 20b, 23a, 23b** und/oder Linsen **11a, 11b** wird man vorzugsweise derart vornehmen, dass bei einer Fundusbetrachtung sich die optischen Achsen **17a, 17b** und **35** immer in der Netzhautgrube schneiden. Selbstverständlich kann auch eine andere Ausrichtung gewählt werden. Die gerade genannte hat sich jedoch für Netzhautbeobachtungen bewährt.

Anstatt LEDs und von diesen getrennt ein Strahlung fokussierendes Element zu verwenden, können auch LEDs mit integrierter Optik verwendet werden. Das integrierte optische Element wird man dann derart auslegen, dass ein nahezu kollimierter Strahl ausgesandt wird. Diese integrierten Systeme (LED + Kollimierung) wird man vorzugsweise in Richtung zum Patientenauge hin versetzt derart anordnen, dass jedes Element über die Lupenlinsenfassung vom Rand unter diese verschiebbar ist. Der Lupenrand trägt in der Regel eine Fassung und wird nicht für die Beobachtung verwendet. Dieser Bereich kann somit raumsparend ausgenützt werden.

Anstelle der LEDs **20a, 20b, 23a** und **23b** können auch Lichtleiter verwendet werden, deren Enden dann an den Orten der LEDs zu liegen kommen. Auch die Lichtleiterenden können analog zu den LEDs verstellt werden. Vorzugsweise wird man die Lichtleiter zu einer gemeinsamen Strahlungsquelle im Handgriff **31** führen. Im Handgriff **31** wird man dann auch eine nicht dargestellte Batterie für die Stromversorgung der Strahlungsquelle unterbringen.

Wird einer der LEDs durch einen Bildaufnahme-Chip ersetzt, so kann dessen aufgenommene Bildinformation (Augennetzhaut, Augenvorderfläche, ...) zu einem Bildschirmgerät zu Betrachtungszwecken oder zu einer Speichereinheit zu Dokumentationszwecken geführt werden. Anstatt nur ein LED zu ersetzen, können selbstverständlich auch zwei einander gegenüberliegende durch den Chip ersetzt werden. Es ist dann eine Stereobetrachtung möglich.

Auf den Handgriff **31** kann verzichtet werden. In diesem Fall wird man die Vorrichtung in ihrer Kontur etwa analog einem Kontaktglas ausbilden. Da nur wenig Flüssigkeit für das Verstellen der Lichtquellen benötigt wird, kann auch ein kleines Reservoir hier vorgesehen werden, aus dem mit einem Knopf am äusseren Rand des "Kontaktglases" eine Flüssigkeitbewegung in und aus dem Schlauch heraus vorgenommen werden kann. Auch eine Knopfbatterie für die LEDs kann untergebracht werden.

Der Handgriff 31 kann auch derart abgewandelt werden, dass er als Halteelement dient, welches an einem Spaltlampengerät aufsteckbar ist. Oftmals haben Spaltlampengeräte eine Einheit, in die ein Linsenhalter einsteckbar ist. Vorzugsweise wird man das Halteelement derart ausbilden, dass es anstelle dieses Linsenhalters einsteckbar ist.

Die Vorrichtung kann jedoch auch am Stirnanschlag für den Patienten mit einer Verlängerung angeordnet werden.

Es kann auch der optische Teil eines Kopfophthalmoskops dahingehend abgeändert werden, dass die Vorrichtung an dessen Stirnband gehalten ist.

Eine zum Auge fixierte Halterung schliesst ein "Verwackeln" bei der Betrachtung aus, was insbesondere von Vorteil ist, wenn Bildaufzeichnungen vorgenommen werden.

Anstelle eines Linsensystems zur Fokussierung und Führung der Beleuchtungsstrahlung kann auch ein Hohlspiegelsystem verwendet werden, welches man bei einer ringförmigen Anordnung der Strahlungsquellen ebenfalls ringförmig ausbilden kann. Werden die spiegelnd reflektierenden Flächen auf einem elastischen Mittel aufgebracht, so kann durch dessen Verformung eine Veränderung des Fokussierkegels und dessen Richtung erreicht werden.

Neben der oben beschriebenen Betrachtung des Augenfundus kann die Vorrichtung auch für die Betrachtung anderer Augenbereiche, auch der Oberfläche, verwendet werden.

## Patentansprüche

1. Vorrichtung **(1)** zur Betrachtung und Beleuchtung des Auges, insbesondere des Augenfundus **(3) gekennzeichnet durch** eine Beleuchtungseinheit **(20a/b, 11a/b; 23a, 23b)** und einer vor dem zu untersuchenden Patientenauge **(5)** zu plazierenden Lupenlinseneinheit **(7),** wobei die Beleuchtungseinheit **(20a/b, 11a/b; 23a, 23b)** derart ausgebildet und angeordnet ist, dass aus ihr austretende Freiraumstrahlung **(9a, 9b)** in der Lupenlinseneinheit **(7)** nicht in den Beobachtungsstrahlengang **(10)** fällt.

2. Vorrichtung **(1)** nach Anspruch 1, **dadurch gekennzeichnet, dass** die Beleuchtungseinheit **(20a/b, 11a/b; 23a, 23b)** derart ausgebildet und angeordnet ist, dass aus ihr austretende Freiraumstrahlung die Lupenlinseneinheit **(7)** nicht durchstrahlt, vorzugsweise die Freiraumstrahlung **(9a, 9b)** ausserhalb der Lupenlinse **(7)** in deren Randbereich bzw. in der diesem benachbarter Umgebung verläuft.

3. Vorrichtung **(1)** nach Anspruch 1 oder 2, **dadurch gekennzeichnet, dass** die Beleuchtungseinheit **(20a/b, 11a/b; 23a, 23b)** wenigstens ein Freiraumstrahlung **(9a, 9b)** aussendendes Element **(20a, 20b)** und eine Fokussiereinrichtung **(11a, 11b)** aufweist, mit der eine Strahlformung der Freiraumstrahlung **(9a, 9b)** vornehmbar ist.

4. Vorrichtung **(1)** nach Anspruch 3, **dadurch gekennzeichnet, dass** der Fokussierkegelwinkel **(15a, 15b)** der Beleuchtungsfreiraumstrahlung **(9a, 9b)** und/oder deren optische Achse **(17a, 17b)** einstellbar ist bzw. sind.

5. Vorrichtung **(1)** nach Anspruch 3 oder 4, **dadurch gekennzeichnet, dass** jedes Freiraumstrahlung **(9a,9b)** aussendendes Element **(20a, 20b)** relativ zur Fokussiereinrichtung **(11a, 11b)** örtlich verstellbar ist, um unterschiedliche Strahlformungen der Freiraumstrahlung **(9a, 9b)** für unterschiedliche Betrachtungsorte im oder am Auge **(5)** zu erreichen.

6. Vorrichtung nach Anspruch 5, **dadurch gekennzeichnet, dass** mehrere Freiraumstrahlung aussendende Elemente annähernd äquidistant ringförmig im Lupenlinsenrandbereich angeordnet sind.

7. Vorrichtung **(1)** nach einem der Ansprüche 3 bis 6, **gekennzeichnet durch** mehrere auf einem elastischen Mittel **(29)** angeordnete, die Freiraumstrahlung **(9a, 9b)** aussendende Elemente **(20a, 20b),** deren örtliche Lage, vorzugsweise **durch** Spannen oder Entlasten eines elastischen Mittels, relativ zur Fokussiereinrichtung **(11a, 11b)** veränderbar ist.

8. Vorrichtung nach einem der Ansprüche 1 bis 7, vorzugsweise nach einem der Ansprüche 3 bis 7, **gekennzeichnet durch** einen Handgriff **(31)** zur Halterung der Lupenlinseneinheit mit Beleuchtungseinheit und eine im Handgriff angeordnete Strahlungsquelle, deren Strahlung die Beleuchtungsstrahlung liefert, wobei vorzugsweise die Strahlungsquelle über Strahlungsleiter zu den Freiraumstrahlung aussendenden Elementen geführt ist.

9. Vorrichtung nach einem der Ansprüche 1 bis 8, **gekennzeichnet durch** wenigstens ein im Randbereich der Lupenlinse angeordnetes Bildaufnahmeelement.
